(19) Europäisches Patentamt
European Patent Office
Office européen des brevets

(11) **EP 4 700 777 A1**

(12) **EUROPEAN PATENT APPLICATION**
published in accordance with Art. 153(4) EPC

(43) Date of publication:
**25.02.2026 Bulletin 2026/09**

(21) Application number: **24768880.7**

(22) Date of filing: **15.05.2024**

(51) International Patent Classification (IPC):
***G16B 5/00*** (2019.01)

(52) Cooperative Patent Classification (CPC):
**G16B 5/00**

(86) International application number:
**PCT/JP2024/018011**

(87) International publication number:
**WO 2025/004586 (02.01.2025 Gazette 2025/01)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **29.06.2023 PCT/JP2023/024219**

(71) Applicant: **Fronteo, Inc.**
**Tokyo 108-0075 (JP)**

(72) Inventors:
• **TOYOSHIBA, Hiroyoshi**
**Tokyo 108-0075 (JP)**

• **TAKAGI, Yuna**
**Tokyo 108-0075 (JP)**
• **HAYASHI, Kazumi**
**Tokyo 108-0075 (JP)**
• **MIYAMOTO, Makoto**
**Tokyo 108-0075 (JP)**
• **SAKAI, Miyuki**
**Tokyo 108-0075 (JP)**
• **MIKAMI, Toshiyuki**
**Tokyo 108-0075 (JP)**
• **SATOMI, Yoshinori**
**Tokyo 108-0075 (JP)**

(74) Representative: **Vossius & Partner**
**Patentanwälte Rechtsanwälte mbB**
**Siebertstrasse 3**
**81675 München (DE)**

(54) **PATHWAY ANALYSIS DEVICE, PATHWAY ANALYSIS METHOD, AND PATHWAY ANALYSIS PROGRAM**

(57)  It is possible to obtain useful insights into drug discovery beyond the scope of known molecular interactions by including a pathway generation unit 11 configured to generate a pathway by applying a minimum flow algorithm based on property information representing a property of a molecule acting on a disease or a symptom, connection information representing a connection relationship between molecules, and similarity information representing a similarity between molecules, and a score computation unit 12 configured to compute a dominance score representing strength of a relationship between a molecule and a disease or symptom targeted for drug discovery by summing flow values assigned to one or more molecules connected immediately below one molecule based on flow value assigned to an individual molecule for molecules included in the generated pathway, and computing a dominance score by effectively utilizing flow values assigned to individual molecules during analysis based on a pathway generated by analysis of an intermolecular relationship beyond the scope of known relationships for an interaction between two molecules.

Fig. 1

**Description**

Technical Field

The present invention relates to a pathway analysis apparatus, a pathway analysis method, and a pathway analysis program, and is particularly suitable for use in a technology for generating a pathway representing an intermolecular interaction as a route map.

Background Art

Conventionally, there has been a known pathway (also referred to as an intermolecular network) representing an intermolecular interaction as a route map. The pathway represents a molecule of a gene, protein, etc. using a symbol such as a circle or a square, and is expressed by connecting symbols with arrows that represent intermolecular interactions. Such visualization of the intermolecular interaction allows easier understanding of life phenomena such that it is possible to investigate which path contains a gene group whose expression level has changed. For example, a pathway is widely used in a field of disease treatment or drug discovery.

Note that there has been a known technology that designates any item such as a pathological event, a drug molecule, a gene, or disease-related information, generates an intermolecular network, which is a necessary range of functional and biosynthetic intermolecular connections by search, and allows estimation of a bio-event in which any biomolecule directly or indirectly involves in expression (for example, see PTL 1).

PTL 1 discloses that an intermolecular network generated by connection search using a biomolecule linkage database is narrowed down by being scored by a combination of relationship code or relationship function code, reliability code, and several other codes (for example, a specific group of biomolecules or a pair of biomolecules is highlighted and displayed on the intermolecular network). In addition, PTL 1 discloses that a desired intermolecular network is found by scoring molecular connections found by combining, alone or in combination, relationship code, relationship function code, reliability code, directivity of an action organ or a biomolecule pair, and other information.

The technology described in PTL 1 can be used to select a molecular-function network highly likely to be related to a disease based on knowledge of, for example, a bio-event or a pathological event characteristically expressed in the disease, or change in quantity of biomolecules, and estimate a molecular mechanism of the disease. In addition, it will be possible to develop drug discovery strategies that answer such questions as which process in a network should be inhibited to effectively treat a specific disease or symptom, which molecule in the network is promising as drug discovery target (protein and other biomolecules targeted in pharmaceutical development), what side effect is expected from that drug discovery target, and what assay system should be used to select drug development candidates to avoid these.

However, the technology described in PTL 1 calculates a score based on code indicating a known relationship between two molecules forming a biomolecule pair, code indicating a level of reliability of direct binding for each biomolecule pair and an experimental method on which direct binding is based, etc., and has problems such as presence of various biases and difficulty in performing analysis beyond the scope of known intermolecular interactions.

PTL 1: WO2003/77159

Summary of Invention

Technical Problem

The invention has been made to solve such problems, and an object of the invention is to make it possible to obtain unbiased and useful insights into drug discovery beyond the scope of known intermolecular interactions.

Solution to Problem

To solve the above-mentioned problems, the invention generates a pathway representing an intermolecular interaction as a route map by applying a minimum flow algorithm based on property information representing a property of a molecule acting on a disease or a symptom, connection information representing a connection relationship between molecules, and similarity information representing a similarity between molecules. Then, for each molecule included in the generated pathway, a dominance score that indicates strength of a relationship between a molecule and a disease or symptom serving as a target of drug discovery is computed based on a flow value assigned to each molecule when the minimum flow algorithm is applied. A dominance score of one molecule is computed based on a flow value assigned to one or more molecules connected immediately below the one molecule.

Advantageous Effects of Invention

According to the invention configured as described above, a pathway is generated by a minimum flow algorithm based on similarity information representing a similarity between molecules in addition to property information representing a property of a molecule and connection information representing a connection relationship between molecules, and a dominance score of a molecule is computed based on a flow value assigned to each molecule when the minimum flow algorithm is applied. For this reason, based on a pathway generated by analyzing an intermolecular relationship beyond the scope of known relationships for an interaction between two molecules, flow values assigned to individual molecules during analysis can be effectively utilized to compute a dominance score that represents strength of a relationship between a molecule and a disease or symptom serving as a target of drug discovery. This dominance score makes it possible to obtain useful insights into drug discovery beyond the scope of known intermolecular interactions.

Brief Description of Drawings

[FIG. 1] FIG. 1 is a block diagram illustrating a functional configuration example of a pathway analysis apparatus according to this embodiment.
[FIG. 2] FIG. 2 is a block diagram illustrating a functional configuration example of a feature vector computation apparatus.
[FIG. 3] FIG. 3 is a diagram illustrating an example of a molecule feature vector.
[FIG. 4] FIG. 4 is a schematic diagram for describing conditions (1) and (2) when a minimum flow algorithm is executed.
[FIG. 5] FIG. 5 is a diagram illustrating an example of a pathway generation method.
[FIG. 6] FIG. 6 is a diagram illustrating an example of computing a dominance score by a score computation unit of this embodiment.
[FIG. 7] FIG. 7 is a diagram for describing a first analysis example.
[FIG. 8] FIG. 8 is a diagram for describing a second analysis example.
[FIG. 9] FIG. 9 is a diagram for describing a third analysis example.
[FIG. 10] FIG. 10 is a diagram for describing a fourth analysis example.
[FIG. 11] FIG. 11 is a block diagram illustrating another functional configuration example of the pathway analysis apparatus according to this embodiment.
[FIG. 12] FIG. 12 is a block diagram illustrating a functional configuration example of a pathway analysis apparatus according to another embodiment.
[FIG. 13] FIG. 13 is a diagram schematically illustrating an example of disease genome analysis performed by a disease genome analysis unit.

Description of Embodiments

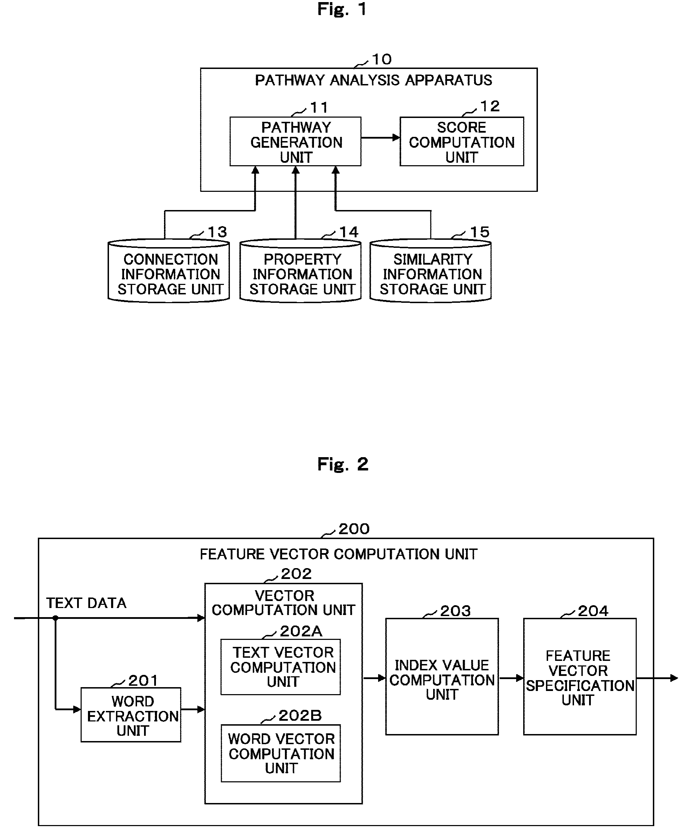

An embodiment of the invention will be described below with reference to the drawings. FIG. 1 is a block diagram illustrating a functional configuration example of a pathway analysis apparatus 10 according to this embodiment. As illustrated in FIG. 1, the pathway analysis apparatus 10 of this embodiment includes, as a functional configuration, a pathway generation unit 11 and a score computation unit 12. In addition, as storage media, a connection information storage unit 13, a property information storage unit 14, and a similarity information storage unit 15 are connected to the pathway analysis apparatus 10 of this embodiment.

The functional blocks 11 and 12 can include any of hardware, a DSP (Digital Signal Processor), and software. For example, the functional blocks 11 and 12 are realized by an operation of a pathway analysis program stored in a storage medium such as a RAM, a ROM, a hard disk, or a semiconductor memory under control of a microcomputer including a CPU, a RAM, a ROM, etc.

The connection information storage unit 13 stores connection information that indicates a connection relationship between molecules. The connection information includes known information such as interactome information. For example, the interactome information is known information related to an intermolecular interaction, such as when an expression level of a certain molecule (gene or protein) increases (or decreases), an expression level of another molecule increases (or decreases) in conjunction with the increase (or decrease) of the expression level of the certain molecule.

Note that this interactome information merely indicates which molecules are related to each other, and does not include information indicating magnitude of a connection relationship. In addition, the interactome information is a collection of information indicating a connection relationship between two molecules, and is not information indicating a sequential connection relationship among three or more molecules.

The property information storage unit 14 stores property information representing a property of a molecule acting on a disease or a symptom. A property of a molecular indicates whether the molecule acts causally or responsively on a disease or a symptom. Causality is a property considered to have a possibility of causing a disease or a symptom due to presence or mutation of the molecule. Responsiveness is a property considered to have a possibility that the molecule may mutate due to onset of a disease or symptom.

Here, when generating a pathway related to a specific disease (hereinafter referred to as a disease pathway), it is possible to use known information recorded in various literature, databases, etc. as information on a plurality of molecules related to the disease and information on properties (causality or responsiveness) of the molecules acting on the disease. In addition, it is possible to use information obtained by estimating a molecule related to a specific disease using a specified algorithm, and estimating a property of a specific molecule acting on the disease using a specified algorithm.

Any algorithm can be used as an algorithm for estimating a molecule related to a disease or for estimating a property of a molecule. For example, a new molecule related to a disease can be estimated by an estimation model trained by machine learning using known information on relevance between a disease and a molecule. In addition, a property of a new molecule can be estimated by an estimation model trained by machine learning using known information on a property of a molecule that acts on a disease.

This description is similarly applied to the case where a pathway related to a specific symptom (hereinafter referred to as a symptom pathway) is generated. That is, known information recorded in various literature, databases, etc. can be used as information on a plurality of molecules related to a symptom and information on properties of these molecules acting on the symptom. In addition, it is possible to use information obtained by estimating a molecule related to a specific symptom using a specified algorithm, and estimating a property of a specific molecule acting on the symptom using a specified algorithm.

The similarity information storage unit 15 stores similarity information representing a similarity between molecules. Here, the similarity information storage unit 15 stores information representing a similarity between respective molecules for a plurality of molecules stored as interactome information in the connection information storage unit 13. The similarity between molecules can be evaluated using various methods. For example, it is possible to apply a method of extracting a predetermined feature quantity for each piece of information related to a plurality of molecules (for example, a description of the molecules, structures or functions of the molecules, etc.) and evaluating a similarity of the feature quantity. As an example, it is possible to use a similarity between pairs of molecules expressed by a fingerprint method.

As another example, it is possible to compute a molecule feature vector by analyzing a plurality of texts (such as literature information) describing information related to a molecule, and use a similarity of the molecule feature vector. The molecule feature vector is data that represents features possessed by a molecule of protein, a gene, etc. (features that can identify the molecule) as a combination of values of a plurality of elements. As an example, a vector that represents a text to which a molecule name included as a word in a plurality of texts contributes and a degree to which the molecule name contributes to the text is used as the molecule feature vector.

A molecule name as a word tends to be used in a text having a description related to a molecule, and not used in a text unrelated to the molecule. In addition, among texts describing a molecule, a text in which a certain molecule name is included as a word is a text describing the molecule, and there is a high possibility that the molecule name is not included in a text describing another type of molecule. In other words, a text in which a molecule name is included as a word tends to vary depending on the type of molecule treated as a theme by the text. Therefore, a vector that represents a text to which a molecule name contributes and a degree to which the molecule name contributes to the text can be used as a feature vector that can identify a molecule.

Such a molecule feature vector can be computed using, for example, an algorithm described in JP6915818B. Hereinafter, a brief description will be given of a method of computing a molecule feature vector using the algorithm described in JP6915818B. FIG. 2 is a block diagram illustrating a functional configuration example of a feature vector computation apparatus 200.

In FIG. 2, a word extraction unit 201 analyzes m texts (m is any integer equal to or greater than 2) and extracts n words (n is any integer equal to or greater than 2) from the m texts. For example, known morphological analysis can be used as text analysis. Note that a plurality of same words may be included in the m texts. In this case, the word extraction unit 201 extracts only one word without extracting the plurality of same words. In other words, the n words extracted by the word extraction unit 201 mean n types of words.

A vector computation unit 202 computes m text vectors and n word vectors from m texts and n words. Here, a text vector computation unit 202A converts each of the m texts to be analyzed by the word extraction unit 201 into a q-dimensional vector according to a predetermined rule to compute m text vectors $d_i\rightarrow$ (i = 1, 2, ..., m) (symbol "$\rightarrow$" indicates being a vector) including q axis components (q is any integer equal to or greater than 2). In addition, a word vector computation unit 202B converts each of the n words extracted by the word extraction unit 201 into a q-dimensional vector according to a predetermined rule to compute n word vectors $w_j\rightarrow$ (j = 1, 2, ..., n) including q axis components. A specific method of computing the text vectors $d_i\rightarrow$ and the word vectors $w_j\rightarrow$ will not be described here.

An index value computation unit 203 computes each of the inner products of the m text vectors $d_i\rightarrow$ and the n word vectors $w_j\rightarrow$ computed by the vector computation unit 202, thereby computing index values reflecting a relationship between the m texts $d_i$ and the n words $w_j$. Here, as shown in the following Equation (1), the index value computation unit 203 obtains the product of a text matrix D having the respective q axis components ($d_{11}$ to $d_{mq}$) of the m text vectors $d_i\rightarrow$ as respective elements and a word matrix W having the respective q axis components ($w_{11}$ to $w_{nq}$) of the n word vectors $w_j\rightarrow$ as respective elements, thereby computing an index value matrix DW having $m \times n$ index values as elements. Here, $W^t$ is the transposed matrix of the word matrix.

[Equation 1]

$$D = \begin{pmatrix} d_{11} & d_{12} & \cdots & d_{1q} \\ d_{21} & d_{22} & \cdots & d_{2q} \\ \cdot & \cdot & \cdot & \cdot \\ \cdot & \cdot & \cdot & \cdot \\ \cdot & \cdot & \cdot & \cdot \\ d_{m1} & d_{m2} & \cdots & d_{mq} \end{pmatrix} \qquad W = \begin{pmatrix} w_{11} & w_{12} & \cdots & w_{1q} \\ w_{21} & w_{22} & \cdots & w_{2q} \\ \cdot & \cdot & \cdot & \cdot \\ \cdot & \cdot & \cdot & \cdot \\ \cdot & \cdot & \cdot & \cdot \\ w_{n1} & w_{m2} & \cdots & w_{mq} \end{pmatrix}$$

$$DW = D * W^t = \begin{pmatrix} dw_{11} & dw_{12} & \cdots & dw_{1n} \\ dw_{21} & dw_{22} & \cdots & dw_{2n} \\ \cdot & \cdot & \cdot & \cdot \\ \cdot & \cdot & \cdot & \cdot \\ \cdot & \cdot & \cdot & \cdot \\ dw_{m1} & dw_{m2} & \cdots & dw_{mn} \end{pmatrix} \quad \cdots (1)$$

Each element of the index value matrix DW computed in this manner may indicate which word contributes to which text and to what extent and which text contributes to which word and to what extent. For example, an element $dw_{12}$ in the first row and the second column may be a value indicating a degree at which the word $w_2$ contributes to a text $d_1$ and may be a value indicating a degree at which the text $d_1$ contributes to a word $w_2$. In this way, each row of the index value matrix DW can be used to evaluate a similarity of a text, and each column can be used to evaluate a similarity of a word.

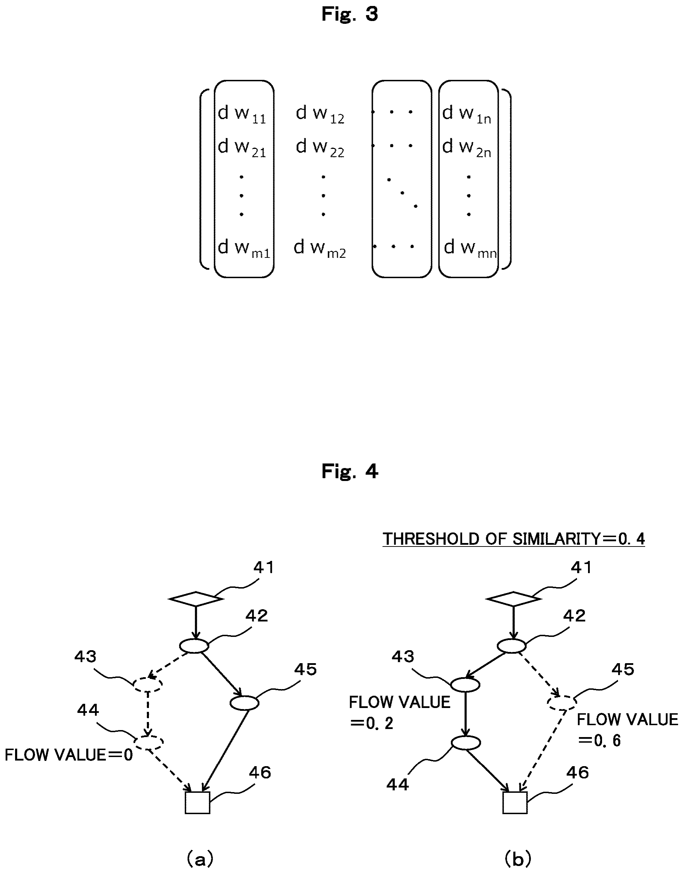

A feature vector specification unit 204 specifies, as a molecule feature vector, a word index value group including m index values for one molecule name for each of a plurality of molecule names among n words. That is, as illustrated in FIG. 3, the feature vector specification unit 204 specifies, as a molecule feature vector corresponding to each molecule name, a word index value group related to a word corresponding to a molecule name among n sets of word index value groups (m index values per column) constituting respective columns of the index value matrix DW.

A similarity of molecule feature vectors can be evaluated in various ways. For example, it is possible to apply a method of extracting a feature quantity for each molecule feature vector using a predetermined function, and evaluating a similarity of the feature quantity. Alternatively, a Euclidean distance or cosine similarity between molecule feature vectors may be used, or an edit distance may be used.

A description will be given by returning to FIG. 1. For example, the pathway generation unit 11 generates a pathway representing an intermolecular interaction as a route map through optimization processing using a minimum flow algorithm based on property information representing a property of a molecule acting on a specific disease or symptom designated by a user, connection information (interactome information) representing a connection relationship between molecules for the designated disease or symptom, and similarity information representing a similarity between molecules. The interactome information, the property information, and the similarity information are used by being read from the connection information storage unit 13, the property information storage unit 14, and the similarity information storage unit 15, respectively.

In this case, the pathway generation unit 11 generates a pathway with a molecule of causality (hereafter, causality molecule) indicated by the property information on an upstream side and a molecule of responsiveness (hereafter, responsiveness molecule) on a downstream side, and by reflecting a connection relationship indicated by the interactome information to place other molecules (hereafter referred to as connectivity molecules) between the causality molecule and the responsiveness molecule, and causing molecules having high similarities as indicated by similarity information to be more likely to be connected to each other. Here, the pathway generation unit 11 sets one start point on the further upstream side of all causality molecules, sets one end point on the further downstream side of all responsiveness molecules, and applies a minimum flow algorithm to a section from the start point to the end point.

Here, when a specific disease is designated by the user, the pathway generation unit 11 generates, as a disease pathway, an intermolecular network in which an intermolecular interaction of a molecule related to the designated disease is represented as a route map. In addition, when a specific symptom is designated by the user, the pathway generation unit 11 generates, as a symptom pathway, an intermolecular network in which an intermolecular interaction of a molecule related to the designated symptom is represented as a route map.

In addition, in this embodiment, the pathway generation unit 11 applies the minimum flow algorithm so as to satisfy at least the following two conditions.

(1) A path is generated by connecting only molecules whose flow values are greater than a minimum threshold and less than a maximum threshold.
(2) A path is generated by connecting only molecules whose flow values are equal to or less than a threshold related to a similarity.

Here, a flow value is an evaluation value computed for each molecule when connecting molecules using the minimum flow algorithm. This evaluation value is computed to satisfy the following conditions.

(A) A flow value of a start point set on an upstream side of the causality molecule is distributed to each of the causality molecule, the connectivity molecule, and the responsiveness molecule in sequence, and a flow value aggregated at an end point set on a downstream side of the responsiveness molecule is the same as the flow value of the start point. A flow value of each molecule is smaller than the flow values of the start point and the end point.
(B) The magnitude of the flow value of each molecule is determined taking into account a similarity between molecules. For example, the flow value is determined based on a predetermined function or calculation algorithm that uses a similarity as a variable. This function or calculation algorithm is designed so that, as the similarity increases, the flow value increases.
(C) The flow value is normalized to a value between 0 and 1. The magnitude of the similarity between molecules is also normalized to a value between 0 and 1. In this case, the flow value may be considered as the similarity between molecules.

With regard to condition (1), for example, a minimum threshold of the flow value can be set to "0". This means that a path including a molecule having a flow value of "0" is not adopted, and any of flow values of respective molecules included in a generated pathway does not become "0". The maximum threshold of the flow value in condition (1) may be different from or the same as a threshold related to a similarity in condition (2). When the values are the same, it means that the maximum threshold of the flow value in condition (1) is defined by the threshold related to the similarity (condition (2) is incorporated into condition (1)).

FIG. 4 is a schematic diagram for describing the above-mentioned conditions (1) and (2). In a path illustrated in FIG. 4, a diamond-shaped node represents a causality molecule, a rectangle-shaped node represents a responsiveness molecule, and an oval-shaped node represents a connectivity molecule (similarly applied to the other figures described below).

As illustrated in FIG. 4(a), it is assumed that two paths can be adopted by the interactome information as a path from a causality molecule 41 to a responsiveness molecule 46. That is, it is possible to adopt a first path of the causality molecule 41 → connectivity molecules 42, 43, and 44 → the responsiveness molecule 46, and a second path of the causality molecule 41 → connectivity molecules 42 and 45 → the responsiveness molecule 46. Here, for example, when a flow value of the connectivity molecule 44 is computed to be zero, the first path is eliminated and the second path is adopted.

In addition, as illustrated in FIG. 4 (b), similarly to 4(a), in a situation where the first path and the second path can be adopted (where the flow value of the connectivity molecule 44 is ≠ 0), it is assumed that a flow value of one connectivity molecule 43 branching off from the connectivity molecule 42 is computed to be 0.2, and a flow value of the other connectivity molecule 45 is computed to be 0.6. Here, when a threshold related to similarity is 0.4, the flow value of the connectivity molecule 45 exceeds an upper limit of the similarity, and thus the second path is eliminated and the first path is adopted. Note that, when the flow values of both connectivity molecules 43 and 45 are smaller than 0.4, both the first and second paths are adopted.

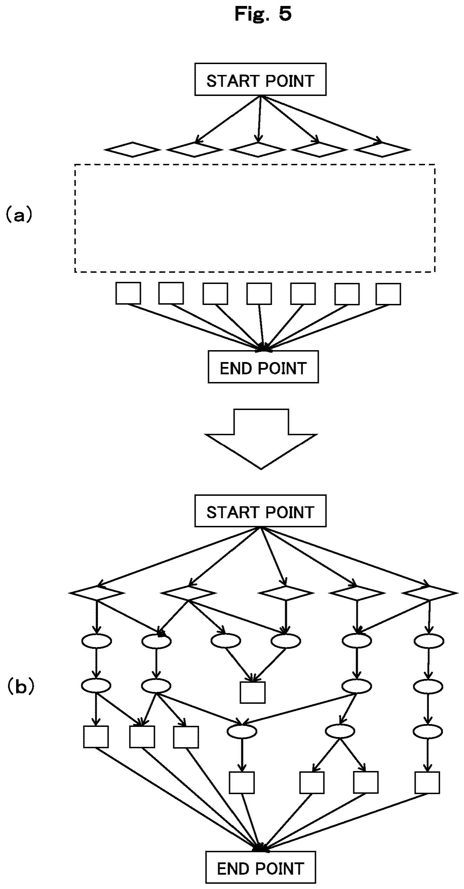

FIG. 5 is a diagram illustrating an example of a pathway generation method by the pathway generation unit 11. First, as illustrated in FIG. 5(a), the pathway generation unit 11 disposes the causality molecule on the upstream side, and disposes the responsiveness molecule on the downstream side. Then, a start point is set on the further upstream side of all causality molecules, and an end point is set on the further downstream side of all responsiveness molecules.

Next, as illustrated in FIG. 5(b), the pathway generation unit 11 applies a minimum flow algorithm to a section from the start point to the end point, and disposes connectivity molecules between causality molecules and responsiveness molecules based on a connection relationship indicated by interactome information, thereby generating a pathway. In this instance, the pathway generation unit 11 computes a flow value of each molecule to satisfy conditions (A) to (C), and performs optimization processing to satisfy conditions (1) and (2) based on the computed flow value.

A description will be given by returning to FIG. 1. The score computation unit 12 computes a dominance score representing strength of a relationship between a molecule and a disease or symptom targeted for drug discovery, based on a flow value assigned to each molecule when applying the minimum flow algorithm, for molecules included in a pathway generated by the pathway generation unit 11. Here, the score computation unit 12 computes a dominance score of one molecule based on a flow value assigned to one or more molecules connected immediately below the one molecule.

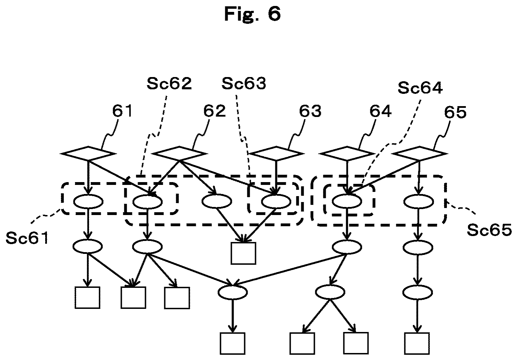

FIG. 6 is a diagram illustrating an example of computing a dominance score by the score computation unit 12 of this embodiment. FIG. 6 illustrates an example of computing dominance scores Sc61 to Sc65 for five causality molecules 61 to 65 included in a pathway. For example, the dominance scores Sc61 and Sc65 of the leftmost causality molecule 61 and the rightmost causality molecule 65 are computed based on flow values assigned to two connectivity molecules connected immediately below the causality molecules 61 and 65, respectively. For example, the sum of the flow values assigned to the two connectivity molecules is computed as the dominance scores Sc61 and Sc65.

In addition, the dominance score Sc62 of the second causality molecule 62 from the left is computed by summing flow values assigned to three connectivity molecules connected immediately below the causality molecule 62. Each of the dominance scores Sc63 and Sc64 of the third and fourth causality molecules 63 and 64 is computed by summing flow values assigned to one connectivity molecule connected immediately below the causality molecules 63 and 64. In this case, the flow value = dominance score.

Here, an example of computing the dominance scores Sc61 to Sc65 of the causality molecules 61 to 65 has been illustrated. Dominance scores of the connectivity molecules are similarly computed. Note that, even though an example of computing, as a dominance score, the sum of flow values assigned to one or more molecules connected immediately below has been described here, the invention is not limited thereto. For example, multiplication or average value of flow values assigned to one or more molecules connected immediately below may be computed as a dominance score.

In addition, a dominance score of each molecule may be computed as a relative value using dominance scores computed in a plurality of pathways. For example, a value obtained by dividing a dominance score of one molecule computed as described above in one pathway by a maximum value of dominance scores of all molecules included in a plurality of pathways may be used as a dominance score of the one molecule.

As described above, in this embodiment, a pathway is generated using a minimum flow algorithm based on similarity information representing similarity between molecules in addition to property information representing a property of a molecule and connection information (interactome information) representing a connection relationship between molecules, and a dominance score of a molecule is computed based on a flow value assigned to each molecule when applying the minimum flow algorithm.

For this reason, based on a pathway generated by analyzing an intermolecular relationship beyond the scope of known relationship about interactions between two molecules, flow values assigned to individual molecules during the analysis can be effectively utilized to compute a dominance score that represents strength of a relationship between a molecule and a disease or symptom targeted for drug discovery. Using this dominance score, it is possible to obtain useful insights into drug discovery beyond the scope of known intermolecular interactions.

Hereinafter, a description will be given of an example of analysis using a dominance score computed as described above.

<First analysis example>

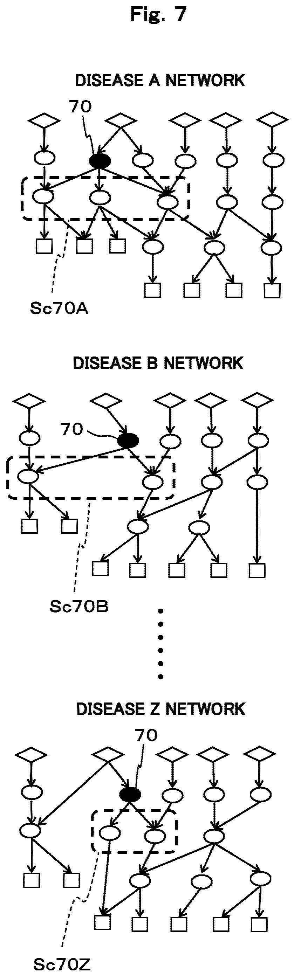

A first analysis example is performing analysis of a pathway from a viewpoint of which disease or symptom is preferably targeted for a certain molecule (for example, a drug discovery candidate gene). FIG. 7 is a diagram for describing the first analysis example.

In the first analysis example, the pathway generation unit 11 generates a plurality of pathways representing, as route maps, intermolecular interactions of related molecules for a plurality of diseases or a plurality of symptoms. FIG. 7 illustrates an example of generating a plurality of disease pathways related to disease A to disease Z.

The score computation unit 12 computes dominance scores Sc70A to Sc70Z of target molecules 70 commonly included in a plurality of pathways for each of the plurality of pathways. Further, the score computation unit 12 may rank the plurality of dominance scores Sc70A to Sc70Z computed for each of the plurality of pathways in descending order of value.

By computing a dominance score of a molecule as in the first analysis example, it is possible to rank a height of possibility that a disease or a symptom for one molecule is a candidate for a target disease or a target symptom based on a dominance score by the dominance score. In addition, it is possible to specify a molecule having a highest dominance score as the target disease or the target symptom.

<Second analysis example>

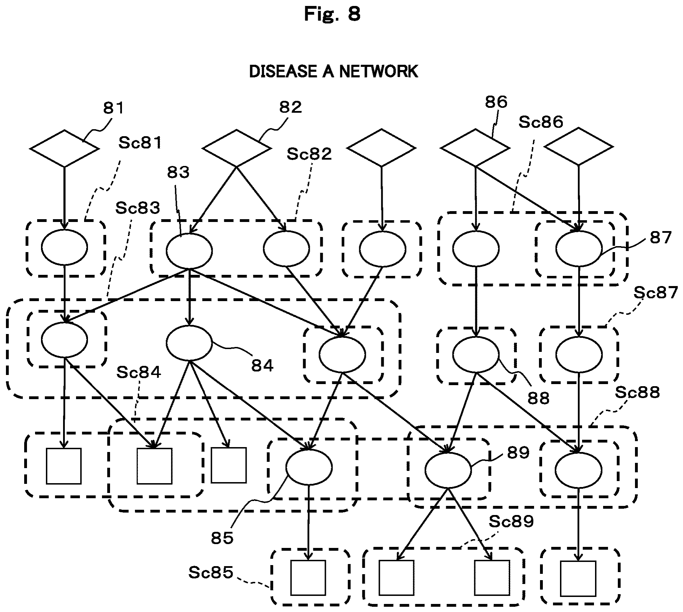

A second analysis example is performing analysis of a pathway from a viewpoint of which molecule is preferably targeted for drug discovery for a certain disease or symptom. FIG. 8 is a diagram for describing the second analysis example.

In the second analysis example, the pathway generation unit 11 generates one pathway that represents, as a route map, an intermolecular interaction of related molecules for one disease or one symptom. FIG. 8 illustrates an example of generating one disease pathway related to disease A.

The score computation unit 12 computes a dominance score for each of a plurality of molecules included in one pathway. FIG. 8 illustrates an example in which a dominance score is computed for each of all causality molecules and all connectivity molecules included in a pathway (for ease of viewing, only some molecules and dominance scores corresponding thereto are labeled 81 to 89 and Sc81 to Sc89). Note that a dominance score may be computed only for a causality molecule or a dominance score may be computed only for a connectivity molecule.

Further, the score computation unit 12 may rank a plurality of dominance scores computed for a plurality of molecules included in one pathway, respectively, in descending order of value.

By computing a dominance score of a molecule as in the second analysis example, it is possible to rank a height of possibility that a certain molecule is a candidate for a target molecule with respect to one disease or symptom by the dominance score. In addition, it is possible to specify a molecule having a highest dominance score as the target molecule.

<Third analysis example>

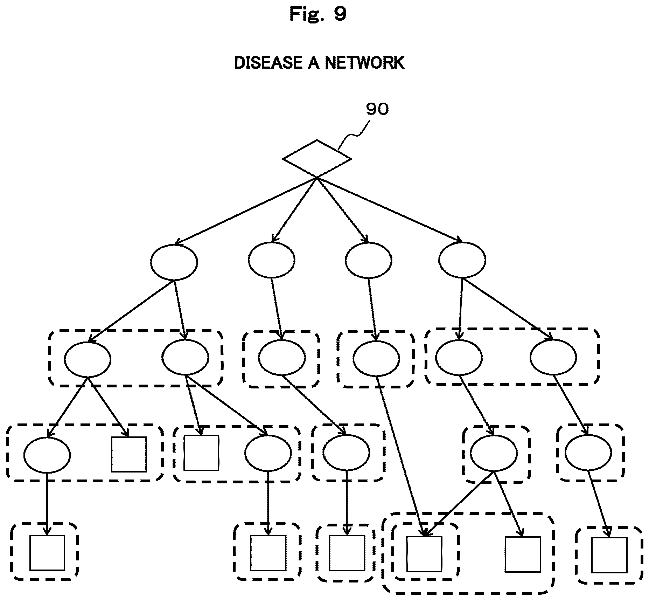

A third analysis example is performing analysis of a pathway from a viewpoint of predicting a suppressor gene for a certain disease or symptom. FIG. 9 is a diagram for describing the third analysis example.

In the third analysis example, the pathway generation unit 11 generates one pathway representing, as a route map, an intermolecular interaction starting from only one causality molecule among molecules related to one disease or one symptom. FIG. 9 illustrates a pathway generated by designating a loss-of-function causality molecule 90 among five causality molecules (◊) included in the pathway of disease A illustrated in FIG. 7, and setting only the causality molecule 90 as a start point and seven responsiveness molecules (□) included in the pathway of disease A illustrated in FIG. 7 as end points.

The score computation unit 12 computes a dominance score for each of a plurality of molecules included in the pathway of disease A, except for the one causality molecule 90. FIG. 9 illustrates an example in which the dominance score is computed for each of all connectivity molecules. Note that it is possible to compute dominance scores for only some of the connectivity molecules (for example, connectivity molecules connected immediately below the causality molecule 90).

Further, the score computation unit 12 may rank a plurality of dominance scores computed for a plurality of molecules included in one pathway, respectively, in descending order of value.

By computing a dominance score of a molecule as in the third analysis example, it is possible to rank a height of possibility that a certain molecule is a candidate for a suppressor molecule with respect to one disease or symptom by the dominance score. Since a suppressor gene is highly likely to be present among molecules having high dominance scores, it is possible to select an analysis candidate molecule as a suppressor based on the dominance scores.

<Fourth analysis example>

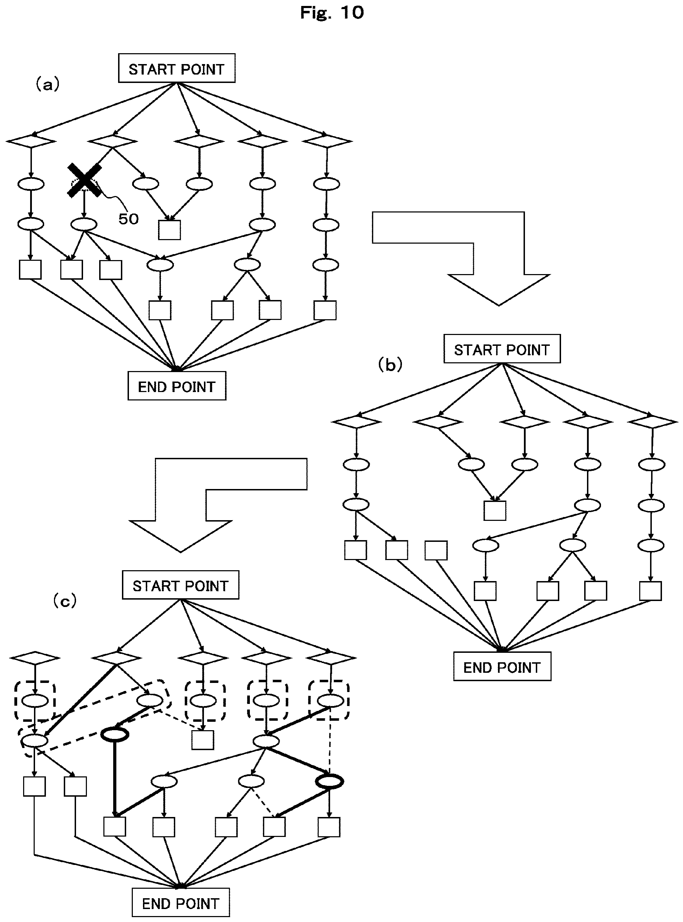

A fourth analysis example is performing analysis of a pathway from a viewpoint of what effect occurs on the entire pathway when a specific molecule is knocked out in the pathway generated once. FIG. 10 is a diagram for describing the fourth analysis example.

FIG. 10 is a diagram illustrating an example of regenerating a pathway by knocking out one connectivity molecule 50 included in a pathway generated once. FIG. 10(a) is an initially generated pathway and illustrates that one connectivity molecule 50 in the pathway is knocked out. A molecule to be knocked out can be arbitrarily designated. For example, it is possible to knock out a molecule that is a target gene for drug discovery.

Knocking out the molecule 50 is equivalent to forcibly setting the flow value of that molecule 50 to zero. Therefore, as illustrated in FIG. 10(b), a path passing through the knocked-out molecule 50 is eliminated. When a path including the knocked-out molecule 50 disappears, the flow value that has been assigned to the path is assigned to another path. In this instance, the pathway generation unit 11 recalculates a flow value of each molecule to satisfy conditions (A) to (C), and re-executes optimization processing based on the recalculated flow value to satisfy conditions (1) and (2).

Therefore, in addition to the path including the knocked-out molecule 50, a path that has been adopted before knockout since the path satisfied conditions (1) and (2) may disappear after knockout since the path no longer satisfies conditions (1) and (2). Conversely, a path that has not been adopted before knockout since the path does not satisfy conditions (1) and (2) may be adopted after knockout since the path satisfies conditions (1) and (2). As a result, the pathway before the knockout illustrated in FIG. 10(a) changes to a pathway illustrated in FIG. 10(c). In FIG. 10(c), a dotted line indicates a disappearing path, and a bold line indicates a newly created path.

The score computation unit 12 computes a dominance score for each of a plurality of molecules included in the pathway regenerated by knocking out the specific molecule 50. FIG. 10(c) illustrates an example in which a dominance score is computed for each of all causality molecules (◊). A dominance score may be further computed for each of all connectivity molecules (oval symbol). The score computation unit 12 may further rank a plurality of dominance scores computed for a plurality of molecules included in the regenerated pathway, respectively, in descending order of value.

In addition, the score computation unit 12 may compute a dominance score for each of the plurality of molecules included in the pathway (pathway of FIG. 10(a)) before knocking out the specific molecule 50, compute a dominance score for each of the plurality of molecules included in the pathway (pathway of FIG. 10(c)) regenerated by knocking out the specific molecule 50, and compute a difference between dominance scores calculated for the same molecule before and after knockout. The score computation unit 12 may further perform ranking based on the magnitude of the computed difference.

A pathway regenerated by knocking out a specific molecule is considered to be a pathway that exhibits drug resistance (treatment resistance) when a drug treatment targeting the specific molecule is administered. By computing the dominance score of the pathway, it is possible to rank a height of possibility of being a candidate for a drug discovery target molecule against a disease exhibiting treatment resistance by the dominance score.

Note that, in the above embodiment, in addition to conditions (1) and (2), one or both of the following conditions (3) and (4) may be added.

(3) A network size of the pathway falls within a preset range. The network size may be, for example, the number of molecules included on a path from a causality molecule to a responsiveness molecule, or the total number of molecules included in the entire pathway.

(4) When a plurality of paths can be adopted from one causality molecule to one responsiveness molecule, a shortest path (the number of included molecules is minimum) is selected therefrom. Alternatively, a path in which the sum of flow values of respective molecules from a causality molecule to a responsiveness molecule is maximum is adopted.

In addition, in the above embodiment, a description has been given on the assumption that the flow value of each molecule is a value determined by taking into account the similarity between molecules. However, the invention is not limited thereto. For example, the flow value of each molecule may be a value computed independently of the similarity between molecules, and the flow value of each molecule may be unequal to the similarity between molecules.

Furthermore, the disease pathway or symptom pathway may be generated using, for example, the algorithm described in JP6915818B.

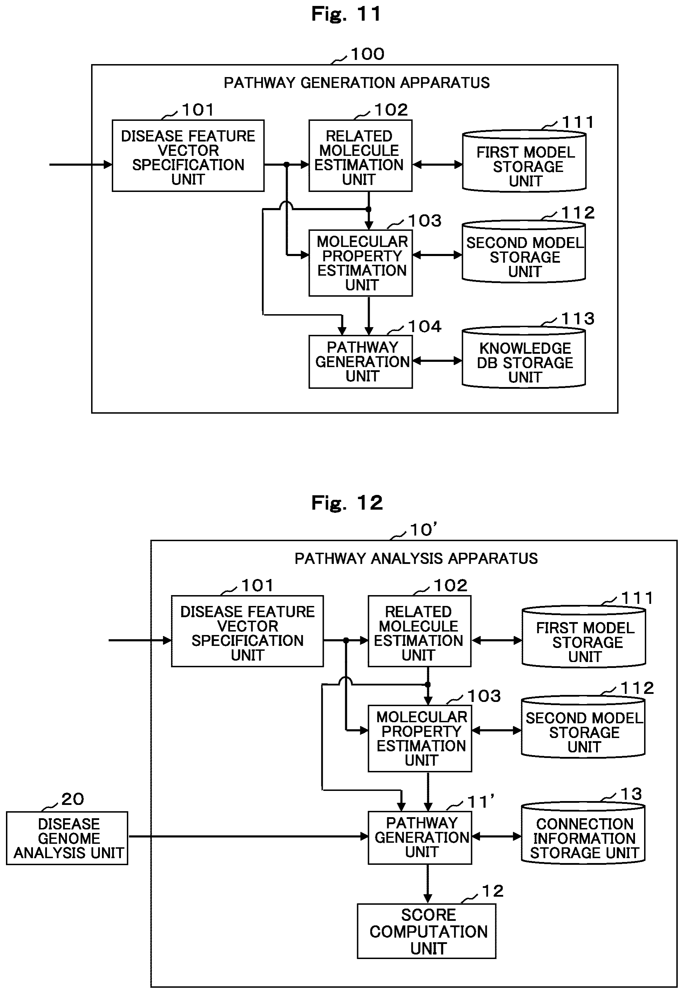

Hereinafter, a brief description will be given of a pathway generation method when using the algorithm described in JP6915818B. FIG. 11 is a block diagram illustrating a functional configuration example of a pathway generation apparatus 100 when the algorithm is used. Note that, here, the case where a disease pathway is generated will be described as an example.

A disease feature vector specification unit 101 specifies a feature vector (hereinafter referred to as a disease feature vector) corresponding to a disease name. The disease feature vector is data representing features of the disease (features that can identify the disease) as a combination of values of a plurality of elements. As an example, a vector representing a text to which a disease name included as a word in a plurality of texts contributes and a degree at which the disease name contributes to the text is used as a disease feature vector.

Such a disease feature vector can be computed, for example, by the feature vector computation apparatus 200 illustrated in FIG. 2. The feature vector computation apparatus 200 receives input of text data related to a text and computes a disease feature vector that reflects a relationship between the text and a word included therein. That is, in the index value matrix DW computed as illustrated in FIG. 3, among n word index value groups included in each column, a word index value group related to a word corresponding to a disease name is specified as a disease feature vector for each disease name.

A related molecule estimation unit 102 inputs a disease feature vector specified by the disease feature vector specification unit 101 to a first trained model stored in advance in a first model storage unit 111, thereby estimating a plurality of molecules associated with a disease. Here, the first trained model is subjected to machine learning so as to output information about a molecule corresponding to a molecule feature vector similar to a disease feature vector when the disease feature vector is input based on a similarity between the disease feature vector and the molecule feature vector.

The feature vector computation apparatus 200 computes a disease feature vector related to a plurality of disease names and computes a molecule feature vector related to a plurality of molecule names. Then, the first trained model is machine-trained in advance using these data sets, and the first trained model trained based on a similarity between the disease feature vector and the molecule feature vector is stored in the first model storage unit 111.

Here, the similarity between the disease feature vector and the molecule feature vector can be evaluated using various methods. For example, it is possible to apply a method of extracting a feature quantity using a predetermined function for each of the disease feature vector and the molecule feature vector and evaluating a similarity of the feature quantity. Alternatively, it is possible to use a Euclidean distance or cosine similarity between the word index value group of the disease feature vector and the word index value group of the molecule feature vector, or it is possible to use an edit distance.

A molecular property estimation unit 103 inputs a disease feature vector specified by the disease feature vector specification unit 101 and a molecule feature vector specified for a plurality of molecules estimated by the related molecule estimation unit 102 to a second trained model stored in a second model storage unit 112, thereby estimating a probability that a molecule acting on the disease is causative or responsive as a property for each of a plurality of molecules presumed to be associated with the disease.

Here, the second trained model is subjected to machine learning to output a probability that a property of a molecule is causative or responsive when a disease feature vector and a molecule feature vector are input using the disease feature vector, the molecule feature vector, and a data set of property information representing the property of the molecule acting on a disease as teacher data.

Using properties of molecules estimated by the molecular property estimation unit 103 and known interactome information indicating a connection relationship between molecules stored in a knowledge DB storage unit 113, a pathway generation unit 104 places a causality molecule on an upstream side and a responsiveness molecule on a downstream side for a plurality of molecules, relevance of which with respect to a disease is estimated by the related molecule estimation unit 102, and reflects a connection relationship indicated by interactome information in other connectivity molecules to dispose the connectivity molecules between the causality molecule and the responsiveness molecule, thereby generating a pathway (intermolecular network) representing an intermolecular interaction as a route map.

In the pathway analysis apparatus 10 illustrated in FIG. 1, when a pathway is generated using the above-described algorithm described in JP6915818B, the pathway generation unit 104 corresponds to the pathway generation unit 11 of FIG. 1, and the knowledge DB storage unit 113 corresponds to the connection information storage unit 13 of FIG. 1. In addition, the pathway generation unit 11 generates a pathway using an estimation result by the molecular property estimation unit 103 instead of the property information storage unit 14 of FIG. 1.

In this instance, for example, the pathway generation unit 11 computes a flow value of each molecule to satisfy conditions (A) to (C), and generates a pathway using a minimum flow algorithm to satisfy conditions (1) and (2) based on the computed flow value by disposing a causality molecule whose probability value estimated by the molecular property estimation unit 103 as causative is greater than a first threshold Th1 on an upstream side of the pathway, disposing a responsiveness molecule whose probability value is less than a second threshold Th2 (Th1 > Th2) on a downstream side of the pathway, and disposing a connectivity molecule whose probability value is greater than or equal to the second threshold Th2 and less than or equal to the first threshold Th1 between the causality molecule and the responsiveness molecule.

Alternatively, the pathway generation unit 11 may dispose a causality molecule whose probability value estimated by the molecular property estimation unit 103 as causative is greater than the first threshold Th1 on the upstream side of the pathway, dispose a responsiveness molecule whose probability value estimated as responsive is greater than a third threshold Th3 (Th1 = Th3 or Th1 ≠ Th3 may be satisfied) on the downstream side of the pathway, and dispose other connectivity molecules between the causality molecule and the responsiveness molecule.

Here, as the similarity used in condition (B) when computing the flow value, instead of the similarity information stored in the similarity information storage unit 15, for example, a value of a similarity between a disease feature vector specified by the related molecule estimation unit 102 and a molecule feature vector may be used. In addition, as a threshold related to the similarity used in condition (2), a threshold related to a similarity between a disease feature vector and a molecule feature vector may be used.

As another example, instead of disposing the causality molecule estimated to be causative by the molecular property estimation unit 103 on the upstream side of the pathway, a gene whose expression level has been found to fluctuate by predetermined disease genome analysis may be disposed on the upstream side as a causality molecule. FIG. 12 is a block diagram illustrating a functional configuration example of a pathway analysis apparatus 10' in this case. In FIG. 12, those given the same reference numerals as reference numerals illustrated in FIG. 1 and FIG. 11 have the same functions, and thus a duplicated description will be omitted here.

In the example illustrated in FIG. 12, the pathway analysis apparatus 10' includes a pathway generation unit 11' instead of the pathway generation unit 104 (corresponding to the pathway generation unit 11) illustrated in FIG. 11. In addition, a disease genome analysis unit 20 is connected to the pathway analysis apparatus 10'. For example, a first information processing apparatus including the pathway analysis apparatus 10' and a second information processing apparatus including the disease genome analysis unit 20 are configured as separate entities, and the first information processing apparatus and the second information processing apparatus are connected via a communication network.

The pathway generation unit 11' of the pathway analysis apparatus 10' acquires information on an analysis result by the disease genome analysis unit 20 via a communication network, and uses the information. Note that it is inessential that the pathway analysis apparatus 10' and the disease genome analysis unit 20 are connected via the communication network. For example, the pathway analysis unit 10' may be configured to acquire the information on the analysis result by the disease genome analysis unit 20 via removable media. In addition, the pathway analysis apparatus 10' may be configured to have a processing function of the disease genome analysis unit 20.

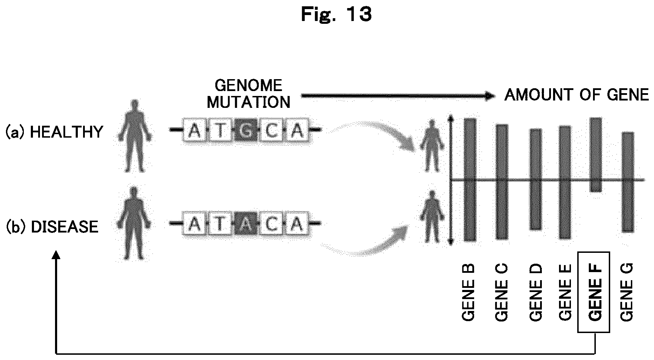

The disease genome analysis unit 20 specifies a gene whose expression level has been found to fluctuate by analyzing a gene locus where a gene expression level fluctuates due to an influence of a disease-associated gene mutation. FIG. 13 is a diagram schematically illustrating an example of disease genome analysis executed by the disease genome analysis unit 20. FIG. 13(a) illustrates results of analyzing a part of a gene sequence of a chromosome extracted from a healthy individual and the amount of each gene included in the chromosome. FIG. 13(b) illustrates results of analyzing a part of a gene sequence of a chromosome extracted from a patient suffering from a specific disease and the amount of each gene included in the chromosome.

FIG. 13 illustrates that a mutation in a certain gene A associated with a disease affects an expression level of another gene F. A gene whose expression level fluctuates in this way is referred to as eGene. The disease genome analysis unit 20 performs disease genome analysis to specify such eGene. As this disease genome analysis, for example, known eQTL (expression Quantitative Trait Locus) analysis can be performed. However, the invention is not limited thereto.

It is known that analyzing eGene, whose expression changes depending on the disease, may lead to search for drug discovery targets. However, even though a huge amount of disease genome information has been obtained by conventional disease genome analysis, there are many diseases for which treatment has not yet been developed since it is difficult to specify a complex disease mechanism. One conceivable reason is that it is difficult to link genetic information with the onset of the disease using a conventional genome analysis method.

To address such a conventional issue, in the pathway generation unit 11' of the pathway analysis apparatus 10' illustrated in FIG. 12, eGene whose expression level has been found to fluctuate by disease genome analysis of the disease genome analysis unit 20 is disposed on the upstream side of the pathway as a causality molecule. In addition, among molecules whose relevance to a disease is estimated by the related molecule estimation unit 102, a molecule estimated to be responsive by the molecular property estimation unit 103 is disposed on the downstream side, and a connectivity molecule other than a molecule estimated to be causative and a molecule estimated to be responsive is disposed between the causality molecule and the responsiveness molecules by reflecting a connection relationship indicated by the interactome information stored in the connection information storage unit 13.

Then, while computing a flow value of each molecule to satisfy conditions (A) to (C) based on the above arrangement, the pathway generation unit 11' generates a pathway by applying a minimum flow algorithm based on the computed flow value to satisfy conditions (1) and (2). In the configuration example illustrated in FIG. 12, the value of the similarity between the disease feature vector specified by the related molecule estimation unit 102 and the molecule feature vector is used as the similarity used in condition (B) when computing the flow value. Note that the similarity information storage unit 15 may be provided, and similarity information stored in the similarity information storage unit 15 may be used.

As described above, by configuring the pathway analysis apparatus 10' as illustrated in FIG. 12 and performing processing, for example, of the second analysis example described above, it is possible to utilize disease genome information analyzed by the disease genome analysis unit 20 to search for a gene serving as a drug discovery target candidate.

Note that, in FIG. 12, a description has been given of an example in which a molecule estimated to be responsive by the molecular property estimation unit 103 is disposed on the downstream side. However, the invention is not limited thereto. For example, the property information storage unit 14 may be provided as in FIG. 1, and a responsive molecule indicated by property information may be disposed on the downstream side of the pathway. When the similarity information storage unit 15 is also provided in addition thereto, it is possible to omit the disease feature vector specification unit 101, the related molecule estimation unit 102, the molecular property estimation unit 103, the first model storage unit 111, and the second model storage unit 112.

In addition, all the embodiments are merely examples of embodiment in carrying out the invention, and the technical scope of the invention should not be construed in a limited manner by the embodiments. That is, the invention can be implemented in various forms without departing from a gist or a main feature thereof.

Reference Signs List

10, 10': pathway analysis apparatus
11, 11': pathway generation unit
12: score computation unit
13: connection information storage unit

14: property information storage unit
15: similarity information storage unit
20: disease genome analysis unit

**Claims**

1. A pathway analysis apparatus **characterized by** comprising:

    a pathway generation unit configured to generate a pathway representing an intermolecular interaction as a route map by applying a minimum flow algorithm based on property information representing a property of a molecule acting on a disease or a symptom, connection information representing a connection relationship between molecules, and similarity information representing a similarity between molecules; and
    a score computation unit configured to compute a dominance score representing strength of a relationship between a molecule and a disease or symptom targeted for drug discovery, based on a flow value assigned to each molecule when applying the minimum flow algorithm, for molecules included in the pathway generated by the pathway generation unit,
    wherein the score computation unit computes the dominance score of one molecule based on a flow value assigned to one or more molecules connected immediately below the one molecule.

2. The pathway analysis apparatus according to claim 1, **characterized in that**:

    the pathway generation unit generates a plurality of pathways representing, as route maps, intermolecular interactions of related molecules for a plurality of diseases or a plurality of symptoms, and
    the score computation unit computes the dominance score of each molecule commonly included in the plurality of pathways for each of the plurality of pathways.

3. The pathway analysis apparatus according to claim 2, **characterized in that** the score computation unit ranks a plurality of dominance scores each calculated for the molecule commonly included in the plurality of pathways.

4. The pathway analysis apparatus according to claim 1, **characterized in that**:

    the pathway generation unit generates one pathway representing, as a route map, an intermolecular interaction of related molecules for one disease or one symptom, and
    the score computation unit computes the dominance score for each of a plurality of molecules included in the one pathway.

5. The pathway analysis apparatus according to claim 4, **characterized in that** the score computation unit ranks a plurality of dominance scores computed for the plurality of molecules included in the one pathway, respectively.

6. The pathway analysis apparatus according to claim 1, **characterized in that**:

    the pathway generation unit generates one pathway representing, as a route map, an intermolecular interaction starting exclusively from one causality molecule among related molecules for one disease or one symptom, and
    the score computation unit computes the dominance score for each of a plurality of molecules included in the one pathway excluding the one causality molecule.

7. The pathway analysis apparatus according to claim 6, **characterized in that** the score computation unit ranks a plurality of dominance scores computed for the plurality of molecules included in the one pathway, respectively.

8. The pathway analysis apparatus according to claim 1, **characterized in that**:

    when a specific molecule is designated to be knocked out from a pathway once generated, the pathway generation unit regenerates the pathway by applying the minimum flow algorithm in a state in which the molecule designated to be knocked out is deleted, and
    the score computation unit computes the dominance score for each of a plurality of molecules included in a pathway regenerated by the pathway generation unit.

9. The pathway analysis apparatus according to claim 8, **characterized in that** the score computation unit ranks a plurality of dominance scores computed for the plurality of molecules included in the regenerated pathway, respectively.

10. The pathway analysis apparatus according to claim 8, **characterized in that** the score computation unit computes the dominance score for each of a plurality of molecules included in the pathway generated once, computes the dominance score for each of a plurality of molecules included in the regenerated pathway, and computes a difference between dominance scores computed before and after knockout for the same molecule.

11. The pathway analysis apparatus according to claim 1, **characterized in that**:

the pathway generation unit is configured to generate the pathway by disposing a causality molecule indicated by the property information on an upstream side and a responsiveness molecule on a downstream side, disposing the other molecules between the causality molecule and the responsiveness molecule by reflecting a connection relationship indicated by the connection information, and applying the minimum flow algorithm based on the disposing, and

by disease genome analysis for analyzing a gene locus where a gene expression level fluctuates due to an influence of a gene mutation associated with the disease, a gene whose expression level has been found to fluctuate is disposed on the upstream side as the causality molecule.

12. The pathway analysis apparatus according to claim 11, **characterized by** further comprising:

a related molecule estimation unit configured to input a disease feature vector specified for a disease to be analyzed to a first trained model, thereby estimating a plurality of molecules related to the disease; and
a molecular property estimation unit configured to input a disease feature vector specified for the disease to be analyzed and a molecule feature vector specified for the plurality of molecules estimated by the related molecule estimation unit to a second trained model, thereby estimating whether a property acting on the disease is causative or responsive for each of the plurality of molecules,
wherein the pathway generation unit disposes a molecule estimated to be responsive by the molecular property estimation unit on the downstream side.

13. A pathway analysis method **characterized by** comprising:

a step of generating, by a pathway generation unit of a computer, a pathway representing an intermolecular interaction as a route map by applying a minimum flow algorithm based on property information representing a property of a molecule acting on a disease or a symptom, connection information representing a connection relationship between molecules, and similarity information representing a similarity between molecules; and
a step of computing, by a score computation unit of the computer, a dominance score representing strength of a relationship between a molecule and a disease or symptom targeted for drug discovery, based on a flow value assigned to each molecule when applying the minimum flow algorithm, for molecules included in the pathway generated by the pathway generation unit,
the dominance score of one molecule is computed based on a flow value assigned to one or more molecules connected immediately below the one molecule.

14. A pathway analysis program **characterized by** causing a computer to function as:

a pathway generation means configured to generate a pathway representing an intermolecular interaction as a route map by applying a minimum flow algorithm based on property information representing a property of a molecule acting on a disease or a symptom, connection information representing a connection relationship between molecules, and similarity information representing a similarity between molecules; and
a score computation means configured to compute a dominance score representing strength of a relationship between a molecule and a disease or symptom targeted for drug discovery, based on a flow value assigned to each molecule when applying the minimum flow algorithm, for molecules included in the pathway generated by the pathway generation means, and to compute the dominance score of one molecule based on a flow value assigned to one or more molecules connected immediately below the one molecule.

**Fig. 1**

**Fig. 2**

## Fig. 3

$$\begin{bmatrix} dw_{11} & dw_{12} & \cdots & dw_{1n} \\ dw_{21} & dw_{22} & \cdots & dw_{2n} \\ \vdots & \vdots & \ddots & \vdots \\ dw_{m1} & dw_{m2} & \cdots & dw_{mn} \end{bmatrix}$$

## Fig. 4

THRESHOLD OF SIMILARITY=0.4

41
42
43
44
45
46
FLOW VALUE=0

(a)

41
42
43
45
44
46
FLOW VALUE =0.2
FLOW VALUE =0.6

(b)

# Fig. 5

(a)

(b)

## Fig. 6

# Fig. 7

## DISEASE A NETWORK

Sc70A

## DISEASE B NETWORK

Sc70B

## DISEASE Z NETWORK

Sc70Z

## Fig. 8

### DISEASE A NETWORK

# Fig. 9

## DISEASE A NETWORK

## Fig. 10

## Fig. 11

~100

**PATHWAY GENERATION APPARATUS**

~101
DISEASE FEATURE VECTOR SPECIFICATION UNIT

~102
RELATED MOLECULE ESTIMATION UNIT

~111
FIRST MODEL STORAGE UNIT

~103
MOLECULAR PROPERTY ESTIMATION UNIT

~112
SECOND MODEL STORAGE UNIT

~104
PATHWAY GENERATION UNIT

~113
KNOWLEDGE DB STORAGE UNIT

## Fig. 12

~10'

**PATHWAY ANALYSIS APPARATUS**

~101
DISEASE FEATURE VECTOR SPECIFICATION UNIT

~102
RELATED MOLECULE ESTIMATION UNIT

~111
FIRST MODEL STORAGE UNIT

~103
MOLECULAR PROPERTY ESTIMATION UNIT

~112
SECOND MODEL STORAGE UNIT

~20
DISEASE GENOME ANALYSIS UNIT

~11'
PATHWAY GENERATION UNIT

~13
CONNECTION INFORMATION STORAGE UNIT

~12
SCORE COMPUTATION UNIT

Fig. 13

## INTERNATIONAL SEARCH REPORT

| International application No. |
|---|
| **PCT/JP2024/018011** |

**A. CLASSIFICATION OF SUBJECT MATTER**

*G16B 5/00*(2019.01)i
FI:  G16B5/00

According to International Patent Classification (IPC) or to both national classification and IPC

**B. FIELDS SEARCHED**

Minimum documentation searched (classification system followed by classification symbols)

G16B5/00-99/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 6915818 B1 (FRONTEO, INC.) 04 August 2021 (2021-08-04)<br>entire text, all drawings | 1-14 |
| A | JP 2005-521929 A (GENOMATICA, INC.) 21 July 2005 (2005-07-21)<br>entire text, all drawings | 1-14 |
| A | CN 111816246 A (SHANGHAI UNIVERSITY) 23 October 2020 (2020-10-23)<br>entire text, all drawings | 1-14 |

☐ Further documents are listed in the continuation of Box C.   ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **11 June 2024** | **18 June 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**INTERNATIONAL SEARCH REPORT**
**Information on patent family members**

International application No.

**PCT/JP2024/018011**

| Patent document cited in search report | | | Publication date (day/month/year) | Patent family member(s) | | | Publication date (day/month/year) |
|---|---|---|---|---|---|---|---|
| JP | 6915818 | B1 | 04 August 2021 | US entire text, all drawings TW | 2023/0122920 202203239 | A1 A | |
| JP | 2005-521929 | A | 21 July 2005 | US entire text, all drawings WO EP CA AU | 2004/0029149 2003/082214 1495321 2480216 2003222128 | A1 A2 A2 A1 A1 | |
| CN | 111816246 | A | 23 October 2020 | (Family: none) | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 200377159 A

- JP 6915818 B